(19) Europäisches Patentamt
European Patent Office
Office européen des brevets

(11) **EP 1 820 499 A1**

(12) **EUROPEAN PATENT APPLICATION**

(43) Date of publication:
**22.08.2007 Bulletin 2007/34**

(51) Int Cl.:
*A61K 31/122* (2006.01)　　*A61K 47/24* (2006.01)
*A61K 36/05* (2006.01)

(21) Application number: **07250654.6**

(22) Date of filing: **16.02.2007**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **21.02.2006 JP 2006044165**

(71) Applicant: **YAMAHA HATSUDOKI KABUSHIKI KAISHA**
**Iwata-shi, Shizuoka 438-8501 (JP)**

(72) Inventors:
• **Murakami, Nagisa**
  **Iwata-shi**
  **Shizuoka 438-8501 (JP)**
• **Ishikura, Masaharu**
  **Iwata-shi**
  **Shizuoka 438-8501 (JP)**

(74) Representative: **Schlich, George William et al**
**Schlich & Co**
**34 New Road**
**Littlehampton**
**West Sussex BN17 5AT (GB)**

(54) **Green algal extract containing astaxanthin having high storage stability**

(57)　The present invention provides means for increasing the storage stability of astaxanthin in a green algal extract, and a green algal extract containing astaxanthin having high storage stability. The present invention provides a green algal extract comprising astaxanthin at a concentration of 0.5 to 20 wt% expressed in terms of free astaxanthin, and at least one phospholipid at a phospholipid concentration of 0.1 to 15 wt%. This green algal extract has an excellent ability to store stably astaxanthin, and even after storage for one week at 60°C it is possible to retain approximately 80% or more of the astaxanthin.

Fig. 1

EP 1 820 499 A1

**Description**

1. Field of the Invention

The present invention relates to astaxanthin-containing green algal extracts wherein the astaxanthin has high storage stability.

2. Description of the Related Art

Astaxanthin, which is a carotenoid imparting a red color, is known to have a potent antioxidative effect. Thus, it is used as a feed for enhancing the color of fish, a pigment in food, cosmetics, a health food product, and the like.
Astaxanthin is chemically synthesized or extracted from naturally occurring products. The naturally occurring astaxanthins are extracted, for example, from *Eucarida* such as euphausiids and *Pandalus borealis,* from *Phaffia* yeast, and from algae. When astaxanthin is extracted from crustaceans, such as euphausiids, *Pandalus borealis,* and crabs, or from *Phaffia* yeast, the yield of astaxanthin is very low because of their low astaxanthin content. For this reason, astaxanthin is generally extracted from algae, e.g., green algae belonging to the genus *Haematococcus,* having a high astaxanthin content.
Astaxanthin is unstable to heat, oxygen, light, and the like, and a culture suspension of algal cells cannot be stored as is. Thus, algal cells containing astaxanthin are ordinarily stored in a dry form (for example, see Japanese Laid-Open Patent Publication Nos. 7-8212 and 2004-129504). However, Japanese Laid-Open Patent Publication Nos. 7-8212 and 2004-129504 do not discuss the stability of astaxanthin itself in the presence of heat, oxygen, or light during drying. For example, according to a study by the inventor of the present invention, it was found that when drying was simply performed using the drying method described in Japanese Laid-Open Patent Publication No. 2004-129504, astaxanthin in the algal cells was decomposed and thus the astaxanthin content was decreased.
Alternatively, astaxanthin may be stored as an extract from the algal cells. The extraction of astaxanthin is often performed immediately after cultivation of the algal cells because astaxanthin is unstable (for example, see Japanese Laid-Open Patent Publication Nos. 9-111139 and 53-38653). The extraction of astaxanthin from green algae is carried out by organic solvent extraction or supercritical extraction, for example.
It has been proposed to use ethanol, acetone, or hexane as a solvent when astaxanthin is extracted from *Haematococcus* algae with an organic solvent. If the extract thus obtained is used as a food additive, then the residual solvent concentration in the extract must be taken into consideration. Another problem is that the extract is also contaminated with the following: the nutrient source in the *Haematococcus* algae culture suspension, chemicals, chlorophylls, phospholipids, sterols, and other materials (see Japanese Laid-Open Patent Publication No. 2004-41147).
In Japanese Laid-Open Patent Publication No. 2004-41147, the extraction of astaxanthin by supercritical extraction is investigated for the purpose of avoiding the shortcomings of astaxanthin extraction by organic solvent extraction. By appropriately selecting an auxiliary solvent, the astaxanthin extracted through supercritical extraction is hardly contaminated with any phospholipids, proteins, chlorophylls, or sterols, which were unavoidable with organic solvent extraction. However, the cost is higher than for organic solvent extraction, and supercritical extraction is not suited for industrial production.
Furthermore, astaxanthin is unstable to heat, oxygen, or light, regardless of any method employed for extraction, and thus it is preferable in the prior art methods that the extract be handled at a low temperature under a nitrogen atmosphere with shielding from light.

SUMMARY OF THE INVENTION

It is an object of the present invention to provide means for increasing the storage stability of astaxanthin in a green algal extract, and to provide a green algal extract containing astaxanthin having a high storage stability.
The present invention is based on the finding that by adjusting to an appropriate level the concentration of phospholipid, which until now has been thought of as an impurity, in a green algal extract containing astaxanthin, the astaxanthin can be further stabilized.
The present invention provides a green algal extract comprising astaxanthin at a concentration of 0.5 to 20 wt% expressed in terms of free (unesterified) astaxanthin, and at least one phospholipid, wherein the phospholipid concentration is 0.1 to 15 wt%.
In a given embodiment, the phospholipid concentration is 1 wt% or more, 2 wt% or more, or 2.5 wt% or more, and the phospholipid concentration is not more than 10 wt%, not more than 7 wt%, or not more than 5 wt%.
In one embodiment, the extract is an extract of a unicellular green alga of the genus *Haematococcus.*
In a further embodiment, the extract is an extract of the green alga *Haematococcus pluvialis.*
In another embodiment, the phospholipid is phosphatidylcholine.

The present invention also provides a method for increasing the storage stability of astaxanthin in a green algal extract, the method comprising:

determining whether the green algal extract contains at least one phospholipid;
adding at least one phospholipid to the green algal extract if it does not contain originally at least one phospholipid; and
adjusting the phospholipid concentration of the green algal extract to between 0.1 to 15 wt%;
wherein the green algal extract contains astaxanthin at a concentration of 0.5 to 20 wt% expressed in terms of free astaxanthin.

The present invention further provides a food product comprising the green algal extract described above.

According to the present invention, the storage stability of astaxanthin in a green algal extract has been increased by adjusting to a specific concentration the amount of phospholipid in a green algal extract containing astaxanthin at a given concentration. Thus, it is possible to store for a long time the astaxanthin as an extract, and since a low temperature is not required for storage, the handling of the extract is simplified.

BRIEF DESCRIPTION OF THE DRAWINGS

Fig. 1 is a graph showing the relationship between the residual rate of astaxanthin and the phospholipid (lecithin) concentration in a green algal extract according to the invention after storage for one week at 60°C.
Fig. 2 is a graph showing the relationship between the residual rate of astaxanthin and the phospholipid (lecithin) concentration in a more diluted green algal extract according to the invention after storage for one week at 60°C.

DESCRIPTION OF THE PREFERRED EMBODIMENTS

Green Algae

There is no particular limitation on the green algae used in the present invention, as long as the green algae can produce astaxanthin. For example, unicellular algae belonging to the genus *Haematococcus* are preferably used. Examples of the preferable green algae include *Haematococcus pluvialis (H. pluvialis), Haematococcus lacustris (H. lacustris), Haematococcus capensis (H capensis), Haematococcus droebakensi (H droebakensi),* and *Haematococcus zimbabwiensis (H zimbabwiensis).*

Examples of *Haematococcus pluvialis (H. pluvialis)* include the NIES144 strain deposited in the Independent Administrative Institution National Institute for Environmental Studies of Japan, the UTEX2505 strain deposited in the Culture Collection of Algae at the University of Texas, U.S.A., and the K0084 strain deposited in the Scandinavian Culture Center for Algae and Protozoa, Botanical Institute, at the University of Copenhagen, Denmark.

Examples of *Haematococcus lacustris* (*H. lacustris*) include the ATCC30402 and ATCC30453 strains deposited in ATCC, the IAM C-392, IAM C-393, IAM C-394, and IAM C-339 strains deposited in the Institute of Applied Microbiology, University of Tokyo, or the UTEX16 and UTEX294 strains.

Examples of *Haematococcus capensis (H. capensis)* include the UTEX LB1023 strain.

Examples of *Haematococcus droebakensi (H. droebakensi)* include the UTEX55 strain.

Examples of *Haematococcus zimbabwiensis (H. zimbabwiensis)* include the UTEX LB1758 strain.

Among these, *Haematococcus pluvialis* is preferably used.

Cultivation of Green Algae

Green algae containing astaxanthin is obtained by cultivating the green algae as described above under such conditions that the green algae can produce astaxanthin. There are no particular limitations regarding the conditions for the production of astaxanthin. For example, it is possible to employ a method in which vegetative cells of the algae are grown in a medium containing a nitrogen source and trace metals under irradiation with light while bubbling carbon dioxide into the medium, then the cells are subjected to at least one of the following stresses: physical stresses due to light irradiation, biological stresses due to nutrient starvation, and chemical substance stresses due to addition of hydrogen peroxide and/or an iron compound, and thereby encystment of the vegetative cells is induced. It is also possible to cultivate the algal cells by adding acetic acid as a carbon source and under dark conditions for vegetative growth, and then encyst the algal cells. The cultivated green algae are collected by centrifugation, washed with water if necessary, and then subjected to the extraction process described in detail below.

Green Algal Extract

In the present invention, "green algal extract" refers to the oil component in the green algae described above, and is the oil component in which astaxanthin has not been subject to concentration or purification. Here, concentration and purification refer to further selectively obtaining astaxanthin from the extracted oil component by separation and purification means usually performed by those skilled in the art, such as chromatography.

There are no particular limitations regarding the means for extracting the oil component, which contains astaxanthin, and means ordinarily used by those skilled in the art may be adopted. For example, it is possible to perform (1) extraction using a solvent simultaneously or after breaking up the green algae cultivated as described above by mechanical crushing (such as using a bead beater), (2) extraction by squeezing after mechanical crushing, or (3) extraction by a combination of (1) and (2). Alternatively, astaxanthin may be extracted by supercritical extraction. Examples of the solvent used for extraction include organic solvents such as chloroform, hexane, acetone, methanol, and ethanol. When a solvent is used for the extraction, the solvent is removed after the extraction by means usually employed by those skilled in the art.

In the organic solvent extraction that is generally employed for astaxanthin extraction, the astaxanthin is extracted together with other components present in the algal cells, neutral lipids, for example. Depending on the type of extraction solvent and the extraction operation employed, the concentration of astaxanthin in the obtained green algal extract may be usually about 0.5 to 20 wt% expressed in terms of free astaxanthin, and more normally about 0.5 to 18 wt%. It should be noted that astaxanthin in the green algal extract is primarily composed of astaxanthin fatty acid monoesters, although this varies on the culture conditions. The concentration of astaxanthin fatty acid diester is about 15 to 30 wt% of the total astaxanthin content, and the concentration of free astaxanthin often is not more than 1 wt%. Throughout the description of this invention, the weight percentage of astaxanthin is expressed in terms of free astaxanthin, although the astaxanthin may be present in esterified form as well as free astaxanthin.

Phospholipids

In the present invention, "phospholipid(s)" refers to one or more phospholipid selected from the group consisting of phosphatidylcholine (lecithin), phosphatidylethanolamine, phosphatidylserine, phosphatidylinositol, phosphatidylglycerol, and sphingomyelin. There are no particular limitations regarding the origin of these phospholipids, and they may be naturally occurring or synthesized chemically. In the present invention, when a phospholipid is added as described below, phosphatidylcholine (lecithin) may be used preferably. The phospholipid may be already present in the algal cells and is extracted together with astaxanthin, or it can be added to the green algal extract separately.

Increase in Storage Stability of Astaxanthin in Green Algal Extract

In the present invention, the method of increasing the storage stability of astaxanthin in a green algal extract comprises a process of determining that the green algal extract contains at least one phospholipids; adding at least one phospholipid to the green algal extract if it does not contain originally at least one phospholipid; and adjusting the phospholipid concentration in the green algal extract to be between 0.1 and 15 wt%.

The adjusted concentration of phospholipid in the green algal extract according to the invention is 0.1 wt% or more, normally 0.2 wt% or more, preferably 0.3 wt% or more, more preferably 0.45 wt% or more, further preferably 1 wt% or more, yet further preferably 2 wt% or more, and even more preferably 2.5 wt% or more, and the adjusted concentration of phospholipid is not more than 15 wt%, preferably not more than 10 wt%, more preferably not more than 7 wt%, and even more preferably not more than 5 wt%. It is not desirable that the proportion (or concentration) of phospholipid in the green algal extract be too high, because precipitation may occur in the extract at low temperatures.

The astaxanthin concentration in green algal extract in which the phospholipid concentration has been adjusted is, in terms of free astaxanthin, 0.5 wt% or more, preferably 2 wt% or more, more preferably 3 wt% or more, yet further preferably 5 wt% or more, and even more preferably 7 wt% or more, and it is not more than approximately 18 wt%, preferably not more than 15 wt%, and more preferably not more than 12 wt%.

The weight ratio of astaxanthin (in terms of free astaxanthin) to phospholipid in the green algal extract is 1:30 to 1:0.005, preferably 1:5 to 1:0.05, and more preferably 1:3 to 1:0.1.

A green algal extract obtained by supercritical extraction contains hardly any phospholipids. For that reason, the concentration of phospholipids is adjusted by adding a suitable amount of phospholipid to such green algal extract. On the other hand, a green algal extract obtained by solvent extraction often contains phospholipids because phospholipids present in the green algae may be extracted together with the astaxanthin. Consequently, the amount of phospholipids to be added is determined in accordance with the amount (or concentration) of phospholipids already present in the green algal extract. In either case, the final phospholipid concentration is adjusted to be within the above range. In the present invention, the process of confirming the phospholipid concentration in the green algal extract is also included within the scope of adjusting the phospholipid concentration. Therefore, by selecting an appropriate extraction solvent, it is possible to obtain a green algal extract containing both astaxanthin and phospholipids at the desired concentrations according to this invention.

To increase the content of phospholipids in the green algal extract obtained by solvent extraction, it is preferable to break up more finely the algal cells in the solvent. By more finely breaking up the green algae in solvent, more phospholipids, which are cell membrane components, are extracted. For breaking up the algal cells, a device such as a bead beater or a homogenizer may be used preferably. As the solvent used for the extraction, ethanol alone can be used preferably, or a combination of ethanol and another organic solvent may be used.

According to this invention, a green algal extract containing astaxanthin having high storage stability astaxanthin is obtained. When this green algal extract is stored for one week at 60°C with shielding from light, astaxanthin remains in the green algal extract in an amount of at least 50%, preferably 75%, more preferably at least 80%, yet further preferably at least 85%, and even more preferably at least 90%, of its initial concentration. Therefore, in a green algal extract in which the phospholipid concentration has been suitably adjusted, the decomposition of astaxanthin, which is thermolabile, is prevented and thus the storage stability of the green algal extract is increased.

The phospholipids are not only components of living organisms, but are also substances used as a food additive, as an emulsifier, and as food. Thus, no particular problems arise from the presence of one or more phospholipid in the green algal extract, and it is not necessary to remove the phospholipid from the green algal extract after storage.

Food Containing Green Algal Extract Containing Astaxanthin Having High Storage Stability

The food of the present invention contains green algal extract in which the concentration of phospholipids has been adjusted to within a predetermined range. This green algal extract can be ingested as food as is, or alternatively may be diluted with a suitable plant oil or the like to obtain a food containing astaxanthin at a predetermined concentration, in order to facilitate adjustment of the amount of astaxanthin ingested as food. In this case, additional phospholipid can be added to increase the storage stability of the astaxanthin. Preferably, the green algal extract in which the storage stability has been increased is used as is, or is diluted and then processed into capsule form. Alternatively, it is also possible to disperse or dissolve the green algal extract in aqueous solution with an emulsifier or a solubilizer, for consumption as a beverage.

Examples

The present invention will be described below by means of examples in which the *Haematococcus pluvialis* K0084 strain is used. However, the present invention is not limited to these examples. In the examples, the amount of astaxanthin, the amount of phospholipid, and the water content were measured according to the following methods.

Measurement of Amount of Astaxanthin

The amount of astaxanthin in the green algal extract was measured as follows. First, a given amount of the green algal extract was suitably diluted with DMSO, and then its absorbance at 492 nm was measured. The astaxanthin concentration (expressed in terms of free astaxanthin) of the diluted solution was calculated using the following equation, and from this calculated value the astaxanthin concentration of the extract before dilution was calculated.

$$\text{Astaxanthin concentration (wt\%)} = (A*100*F)/(W*2085)$$

where: A is the absorbance at 492 nm of the diluted solution (1 cm optical path length);
F is the dilution factor of the diluted solution; and
W is the weight (g) of the diluted solution.

Measurement of Amount of Phospholipid (lecithin)

The amount of lecithin is calculated by multiplying by 26.03 the measured value (the amount of phosphorous) obtained by the molybdenum blue method (for example, the phosphorous assay using phosphomolybdic acid described in "Methods of Analysis in Health Sciences 2005" edited by The Pharmaceutical Society of Japan, published by Kanehara & Co., Ltd.), which is generally used for assaying phosphorus.

Measurement of Water Content

The water content was measured by an atmospheric oven drying method (such as the method described in "Guidelines for Food Sanitation Testing: Physics and Chemistry Volume" edited by Ministry of Health, Labour and Welfare, Japan Food Hygiene Association, 2005). In other words, a weighing container and its lid are placed in a constant temperature drying oven that has been adjusted to a predetermined temperature, and after heating for one hour, the container with the lid is moved into a desiccator. The weighing container with the lid is allowed to cool until its temperature reaches room temperature, then immediately the weight of the container with the lid is measured on the order of 0.1 mg. Then, the operation of heating, cooling, and weighing of the container with the lid is repeated until a constant weight ($W_0$ g) is reached. Next, 1 to 2 g of sample are quickly taken out and spread out flat on the weighing container, the lid is closed, and the weight thereof is accurately measured ($W_1$ g). The weighing container is placed in the constant temperature drying oven while the lid is shifted aside. Once the temperature of the constant temperature drying oven has reached 105°C, then, after three hours of drying, the lid is quickly placed on the weighing container and closed in the drying oven, and the container with the lid is moved into the desiccator and allowed to cool. The weight is measured immediately after the temperature returns to room temperature ($W_2$ g). The water content of the sample is calculated by the following equation.

$$\text{Water content in sample (\%)} = (W_1 - W_2)/(W_1 - W_0) \times 100$$

Example 1: Preparation of Green Algal Extract - 1

The *Haematococcus pluvialis* K0084 strain (hereinafter referred to simply as the K0084 strain), which produces astaxanthin, was used. One liter of the MBG-11 medium containing the components shown in Table 1 below was placed in a 1.5-L closed flat culture flask with a 25 mm light path, and the K0084 strain was inoculated into the medium at an initial concentration of 0.6 g/L.

Table 1

| MBG- 11 | |
| --- | --- |
| Components | g/L |
| $NaNO_3$ | 0.41 |
| $K_2HPO_4$ | 0.04 |
| $MgSO_4 \cdot 7H_2O$ | 0.075 |
| $CaCl_2 \cdot 2H_2O$ | 0.036 |
| Citric acid (anhydrous) | 0.006 |
| Ammonium iron (III) citrate | 0.006 |
| $EDTA \cdot 2Na$ | 0.001 |
| $Na_2CO_3$ | 0.02 |
| $CuSO_4 \cdot 5H_2O$ | 0.00286 |
| $H_3BO_4$ | 0.00181 |
| $MnCl_2 \cdot 4H_2O$ | 0.00022 |
| $ZnSO_4 \cdot 7H_2O$ | 0.00008 |
| $Na_2MoO_4$ | 0.000021 |
| $Co(NO_3)_2 \cdot 6H_2O$ | 0.000000494 |

While bubbling a gas containing 3 vol% $CO_2$ into the medium at a rate of 600 mL/min (i.e., 0.6 vvm), the culture temperature was adjusted to 25°C and its pH adjusted to between 6 and 8, and the K0084 strain was cultivated for five days under the following conditions of light irradiation. A white fluorescent light (FL40SSW/37, made by Matsushita Electric Industrial Co., Ltd.) was used as the light source. The intensity of the light irradiation was adjusted so that the photosynthetically active photon flux density (PPFD) in the light-receiving direction of the culture flask, which is measured using the LiCor-190SA planar quantum sensor, is 100 $\mu$mol-p/m$^2$s. The K0084 strain after the cultivation was changed in color from green to brown or blackish brown, and was confirmed to have been encysted.

Next, the same medium (MBG-11 medium) was placed in the same flat culture flask as above, and the encysted K0084 strain was inoculated into the medium at an initial concentration of 0.6 g/L, and cultivated for 200 hours under the same culture conditions as described above.

The green algae obtained were collected by centrifugation, washed with water, and placed into a bead beater. Hexane was added thereto, and the green algae were broken up by the bead beater for five minutes. By finely breaking up the green algae in solvent in this manner, more lecithin, which is a cell membrane component, is extracted. The obtained mixture was separated into a supernatant and a precipitate by centrifugation, and the supernatant was collected. Hexane was once again added to the precipitate and the same procedure as above was repeated until the color of the precipitate became almost completely white. The collected hexane fractions were combined, and the hexane was removed to give a green algal extract. The concentration of astaxanthin in the obtained green algal extract was 9 wt% expressed in terms of free astaxanthin, the phospholipid concentration was 0.9 wt%, and the water content was 0.2 wt%.

Example 2: Examination of Phospholipid Concentration - 1

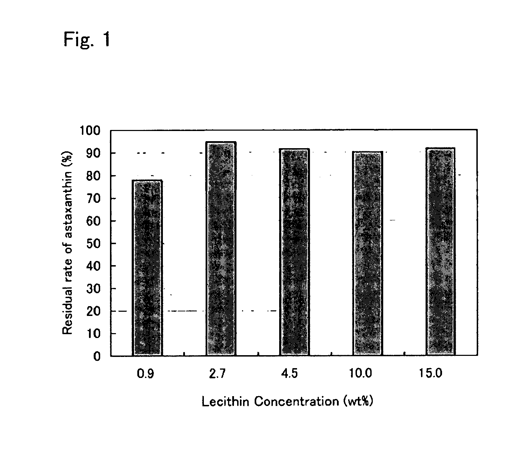

Samples were prepared by adding phospholipid (lecithin from soybean, made by Wako Pure Chemical Industries Ltd.) to the green algal extract obtained in Example 1 (phospholipid: 0.9 wt%; astaxanthin: 9 wt%) so that the phospholipid concentration was 2.7 wt%, 4.5 wt%, 10 wt%, or 15 wt%, respectively. Approximately 500 mg of each sample was placed in each of two 10-mL vials, and the initial concentration of astaxanthin was measured as described above. Then, these vials were placed in an incubator set to 60°C. After storage in the incubator for one week, the astaxanthin concentration was measured again, and the residual rate was calculated. The results are shown in Fig. 1.

As seen from Fig. 1, when the green algal extract contained phospholipid at a concentration of 2.7 wt%, 90% or more of the astaxanthin remained even after one week of storage at 60°C, which is a severe condition, and when the green algal extract contained phospholipid at a concentration of 0.9 wt%, at least 75% of the astaxanthin remained. In particular, it is found that when phospholipids are added to the green algal extract, the residual rate of astaxanthin is very high.

Example 3: Examination of Phospholipid Concentration - 2

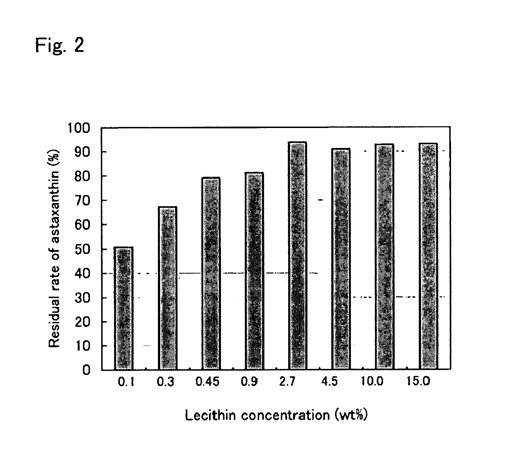

A portion of the green algal extract obtained in Example 1 was diluted by a factor of two with soybean oil (made by The Nisshin OilliO Group Ltd.) to give a diluted extract containing astaxanthin at a concentration of 4.5 wt% expressed in terms of free astaxanthin. Samples were prepared from the diluted green algal extract (phospholipid: 0.45 wt%) by adding a phospholipid (lecithin from soybean, made by Wako Pure Chemical Industries Ltd.) so that the phospholipid concentration was 0.9 wt%, 2.7 wt%, 4.5 wt%, 10 wt%, or 15 wt%, respectively. Other portions of the green algal extract obtained in Example 1 were diluted by a factor of three and by a factor of nine, respectively, with soybean oil to give diluted extracts in which the phospholipid concentration was 0.3 wt% (astaxanthin: 3 wt%) and 0.1 wt% (astaxanthin: 1 wt%), respectively. Approximately 500 mg of each sample was placed in each of two 10-mL vials, and the initial concentration of the astaxanthin was measured as described above. Then, these vials were placed in an incubator set at 60°C. After storage in the incubator for one week, the astaxanthin concentration was measured again, and the residual rate was calculated. The results are shown in Fig. 2.

As seen from Fig. 2, when the green algal extract contained phospholipid at a concentration of 0.45 wt% or more, then approximately 80% or more of the astaxanthin remains even after one week of storage at 60°C, which is a severe condition. On the other hand, the lower the phospholipid concentration, the greater the decrease in the residual rate of astaxanthin. It should be noted that the same test was performed for the green algal extract containing astaxanthin at a concentration of 12 wt% expressed in terms of free astaxanthin, and the same results were obtained.

Comparative Example 1: Examination of Stability of Astaxanthin in a Sample that does not Contain Phospholipids

The encysted K0084 strain obtained by cultivation employing the same procedure as in Example 1 was collected by centrifugation. Then, 5 g of dry algal cells were mixed with an equal amount of diatomaceous earth, broken up by a mortar and a pestle, and then subject to supercritical extraction using the supercritical $CO_2$ extraction device "Spe-ed SFE" (Asahi Life Science). The extraction was performed at a pressure of 400 bar and at an extraction temperature of 50°C while supplying supercritical $CO_2$ at a flow rate of 1 L/min. The astaxanthin concentration in the supercritical extract obtained was 30 wt% in terms of free astaxanthin, and the phospholipids concentration was below the detectable limit (0.01 wt%).

Approximately 500 mg of the obtained supercritical extract was placed in each of two 10-mL vials, and the initial concentration of the astaxanthin was measured as described above. Then, these vials were placed in an incubator set to 60°C. After storage in the incubator for one week, the astaxanthin concentration was measured again. The residual rate of astaxanthin was found to be 0.2%, indicating that the stability of astaxanthin in this extract was extremely low.

Example 4: Examination of Stability to UV Irradiation

A portion of the green algal extract obtained in Example 1 was diluted by a factor of two with olive oil (made by The Nisshin OilliO Group Ltd.) to give a dilute extract containing astaxanthin at a concentration of 4.5 wt% in terms of free astaxanthin. Samples were prepared by adding phospholipid (lecithin from soybean, made by Wako Pure Chemical Industries Ltd.) to the green algal extract (phospholipid: 0.9 wt%; astaxanthin: 9 wt%) and the dilute extract (phospholipid: 0.45 wt%; astaxanthin: 4.5 wt%) so that the phospholipid concentration was 0.9 wt%, 2.7 wt%, 4.5 wt%, 10 wt%, or 15 wt%, respectively. Approximately 500 mg of each sample were placed in each of two 10-mL vials. Then, these vials were placed on a UV irradiation device (desk-top irradiation device DT-35LP; made by ATTO Corporation), and irradiated at 365 nm for one week. After one week of storage, the astaxanthin concentration was measured. The residual rate of astaxanthin was high (approximately 80% or more remaining) in every case where a phospholipid was added, and the same tendency as in Examples 2 and 3 was observed.

Example 5: Preparation of Green Algal Extract - 2

The green algal extract was obtained in the same manner as in Example 1, except that ethanol was used in place of hexane as the extraction solvent. The astaxanthin concentration in the obtained green algal extract was 8.1 wt% expressed in terms of free astaxanthin, the phospholipid concentration was 1.7 wt%, and the water content was 0.3 wt%. Then, this vial was placed in an incubator set to 60°C and stored for one week, thereafter the astaxanthin concentration was measured again. The residual rate of astaxanthin was found to be high (85%).

According to the present invention, an astaxanthin-containing green algal extract wherein the astaxanthin has high storage stability is provided by adjusting to specified levels the concentration of phospholipid in the green algal extract. As a result, it is possible to store astaxanthin as an extract for long periods, and the handling of the extract is simplified because low temperatures are not required.

**Claims**

1.   A green algal extract comprising astaxanthin at a concentration of 0.5 to 20 wt% expressed in terms of free astaxanthin, and at least one phospholipid, wherein the phospholipid concentration is 0.1 to 15 wt%.

2.   The green algal extract of claim 1, wherein the phospholipid concentration is 1 wt% or more.

3.   The green algal extract of claim 2, wherein the phospholipid concentration is 2 wt% or more.

4.   The green algal extract of claim 3, wherein the phospholipid concentration is 2.5 wt% or more.

5.   The green algal extract of any one of claims 1 to 4, wherein the phospholipid concentration is not more than 10 wt%.

6.   The green algal extract of claim 5, wherein the phospholipid concentration is not more than 7 wt%.

7.   The green algal extract of claim 6, wherein the phospholipid concentration is not more than 5 wt%.

8.   The green algal extract of any one of claims 1 to 7, which is an extract of a unicellular green alga of the genus *Haematococcus.*

9.   The green algal extract of claim 8, which is an extract of the green alga *Haematococcus pluvialis.*

10.  The green algal extract of any one of claims 1 to 9, wherein the phospholipid is phosphatidylcholine.

11.  A method for increasing the storage stability of astaxanthin in a green algal extract, comprising:

    determining that the green algal extract contains at least one phospholipids;
    adding at least one phospholipid to the green algal extract if it does not contain originally at least one phospholipid;

and

adjusting the phospholipid concentration of the green algal extract to be between 0.1 to 15 wt%;

wherein the green algal extract contains astaxanthin at a concentration of 0.5 to 20 wt% expressed in terms of free astaxanthin.

12. A food product comprising the green algal extract of any one of claims 1 to 10.

Fig. 1

Fig. 2

**European Patent Office**

**EUROPEAN SEARCH REPORT**

Application Number

EP 07 25 0654

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/112767 A (ADVANCED BIONUTRITON CORP [US]; HAREL MOTI [US]; PIECHOCKI JOHN [US];) 29 December 2004 (2004-12-29) * claims 1-34 * | 1-12 | INV. A61K31/122 A61K47/24 A61K36/05 |
| X | WO 02/064110 A (YISSUM RES DEV CO [IL]; BARENHOLZ YECHEZKEL [IL]; DIMINSKY DVORAH [IL]) 22 August 2002 (2002-08-22) * claims 1-49 * | 1-10,12 | |
| X | JP 08 245335 A (ITANO REITOU KK) 24 September 1996 (1996-09-24) * the whole document * | 1-7,10 | |
| A | WO 03/102116 A (PHARES PHARMACEUTICAL RES N V [NL]; LEIGH STEVE [CH]; LEIGH MATHEW LOU) 11 December 2003 (2003-12-11) * example 13 * * claims 1-17 * * page 5, line 15 - line 33 * | 1-12 | |
| A | EP 0 467 795 A (PATRINOVE [FR]; TEXINFINE SA [FR]) 22 January 1992 (1992-01-22) * claims 1,6-9,13-17 * | 1-12 | TECHNICAL FIELDS SEARCHED (IPC) A61K |

The present search report has been drawn up for all claims

| Place of search | Date of completion of the search | Examiner |
|---|---|---|
| Berlin | 11 May 2007 | Siatou, Evangelia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 25 0654

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2007

| Patent document cited in search report | | Publication date | Patent family member(s) | | Publication date |
|---|---|---|---|---|---|
| WO 2004112767 | A | 29-12-2004 | CA<br>EP | 2529055 A1<br>1633335 A1 | 29-12-2004<br>15-03-2006 |
| WO 02064110 | A | 22-08-2002 | CA<br>EP<br>JP | 2437578 A1<br>1361863 A2<br>2004518707 T | 22-08-2002<br>19-11-2003<br>24-06-2004 |
| JP 8245335 | A | 24-09-1996 | NONE | | |
| WO 03102116 | A | 11-12-2003 | AU<br>CA<br>CN<br>IS<br>JP<br>NZ<br>US | 2003242589 A1<br>2486424 A1<br>1656178 A<br>7549 A<br>2005528448 T<br>536655 A<br>2005260145 A1 | 19-12-2003<br>11-12-2003<br>17-08-2005<br>24-11-2004<br>22-09-2005<br>29-09-2006<br>24-11-2005 |
| EP 0467795 | A | 22-01-1992 | AU<br>FR<br>JP<br>ZA | 8021891 A<br>2664164 A1<br>5025036 A<br>9105121 A | 09-01-1992<br>10-01-1992<br>02-02-1993<br>27-05-1992 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7008212 A
- JP 2004129504 A
- JP 9111139 A
- JP 53038653 A
- JP 2004041147 A

**Non-patent literature cited in the description**

- Methods of Analysis in Health Sciences. Kanehara & Co., Ltd, 2005
- Guidelines for Food Sanitation Testing: Physics and Chemistry Volume. Japan Food Hygiene Association, 2005